# EUROPEAN PATENT SPECIFICATION

(11) **EP 2 283 823 B1**
(45) Date of publication and mention of the grant of the patent: **11.07.2012**
(21) Application number: 09009791.6
(22) Date of filing: 29.07.2009
(51) Int. Cl.: A61K 9/20, A61K 9/16

(54) **Effervescent formulations comprising a phosphonate and methods for their preparation**
Efferveszenzformulierungen mit einem Phosphonat und Herstellungsverfahren dafür
Formules effervescentes comportant un phosphonat et leurs procédés de préparation

(43) Date of publication of application: 16.02.2011
(73) Proprietor: Uni-Pharma Kleon Tsetis Pharmaceutical Laboratories S.A., 145 64 Kifissia (GR); Tseti, Ioulia, 145 61 Kifissia (GR)
(72) Inventor: Tseti, Ioulia, 145 61 Kifissia (GR)
(74) Representative: Wibbelmann, Jobst

(56) References cited:
- WO-A-2004/035004
- WO-A-2007/013119

## Description

### Field of the invention

The present invention describes an effervescent composition and a process for preparing said effervescent composition, under two presentations, i.e. effervescent granules and effervescent tablets, in which therapeutic doses of biphosphonates or their pharmaceutically acceptable salts, preferably alendronate or sodium alendronate, are incorporated, with the intention of treatment of bone resorption-associated conditions such as osteoporosis, or Paget's disease.

### Background of the invention

Bone resorption is a physiological process through which bone tissue is continually renewed and repaired. A multinuclear group of cells called osteoclasts are responsible for causing bone loss, by resorbing old or damaged bone, while another group of cells called osteoblasts have the capacity to synthesize a new bone matrix. Quite a variety of disorders in humans and other mammals are attributed to abnormal bone resorption, with some being osteoporosis, Paget's disease, periprosthetic bone loss or osteolysis, metastatic bone disease, hypercalcemia of malignancy and arthritides.

Out of these disorders, osteoporosis has by far the highest incidence. In its most frequent manifestation it is presented in postmenopausal women and leads to defective skeletal function caused by low bone mass and deterioration of bone quality in terms of microarchitecture of the bone tissue. In patients suffering from osteoporosis, the bone resorption rate exceeds the bone formation one, thus rendering the bone with weakened mechanical strength and much more susceptible to fracture even under stress that would otherwise be tolerated by the normal bone.

Treatment of osteoporosis, which like all other bone tissue loss associated disorders is a chronic condition, is based on inhibition of bone resorption. It is well known that biphosphonates, such as alendronate, which are synthetic analogues of pyrophosphates (natural regulators of bone turnover) act as selective inhibitors of osteoclastic bone resorption, thus making them important therapeutic agents in the treatment and/or prevention of osteoporosis and all other generalized or localized bone disorders caused by or associated with abnormal bone resorption.

Per os administration of biphosphonate compounds or their pharmaceutically acceptable salts for the treatment of osteoporosis and like disorders, nevertheless, suffers from a major drawback: effervescent delivery systems need to be produced in a humidity controlled environment in order to mitigate the risk of undesirable triggering of effervescence by reaction of the acid with the alkaline (carbonate source) component and lack of exposure to humidity must be guaranteed throughout the shelf-life of the product, for quality assurance purposes.

It is thus self evident that humidity control is a critical quality assurance factor and an efficient method for addressing the issue, besides monitoring and adjustment of environmental conditions at the production site, which builds up cost and complicates the production process, is needed.

In the prior art, effervescent formulations regarding preventing humidity-induced disintegration are described, for example using dried zeolite or silica gel as moisture absorbing desiccant (US 2004/0127388 A1) or using protective layers such as polydimethylsiloxane (US 2002/0127184 A1).

Until now, in the prior art using divalent metal salts, particularly magnesium sulfate, as moisture absorbent in effervescent formulations comprising biphosphonates or their pharmaceutically acceptable salts was considered to be unsuitable, as especially divalent cations can be sequestered by biphosphonates or their pharmaceutically acceptable salts, particularly alendronate or sodium alendronate, rendering the biphosphonate or its pharmaceutically acceptable salts less bioavailable. For this reason, WO 2004/035004 A2 discloses effervescent composition comprising biphophonates with the benefit of no polyvalent metal ions as Mg²⁺ or Ca²⁺being present.

Surprisingly, it has now been found that the divalent metal salt magnesium sulfate can be used as humidity moderator with the inclusion of a dual system of excipients to the herein described formulation; magnesium sulfate, which acts as a humidity moderator and a divalent cation scavenger as defined in claim 1, preferably fumaric acid in a sufficient quantity. The divalent cation scavenger acts by sequestering the divalend cations to prevent that these cations are sequestered by the biphosphonate or its pharmaceutically acceptable salt, preferably alendronate or sodium alendronate. The incorporation of this excipient system allows alleviating the need for humidity environmental monitoring during production and provides significant cost benefits.

### Detailed description of the invention

The present invention describes an effervescent composition and a process for preparing an effervescent composition, under two presentations, i.e. effervescent granules and effervescent tablets, in which therapeutic doses of biphosphonates or their pharmaceutically acceptable salts, preferably alendronate or sodium alendronate, are incorporated with the intention of treatment of bone resorption-associated conditions such as osteoporosis, or Paget's disease.
The effervescent composition according to the invention can comprise any suitable amount of the biphosphonate in order to achieve the desired pharmaceutical effect.

Typically, about 0.1% by weight or more biphosphonate, based on the total weight of the composition, may be present. The effervescent composition may comprise about 0.25% to about 33.3% biphosphonate, based on the total weight of the composition. In some embodiments, about 1.5% or more and about 30% or less biphosphonate, based on the total weight of the composition, may be present.

The effervescent composition typically is administered to a mammal, including a human being, following a continuous dosing schedule. A continuous schedule may be daily, once weekly, twice weekly, etc.

The actual amount of biphosphonate present in the composition may depend in part on the specific biphosphonate and the dosing schedule chosen. In the case of alendronate, the amount of biphosphonate is typically about 1 mg to 500 mg, preferably 5 mg to 200 mg.

The biphosphonate can be any suitable pharmaceutically active biphosphonate. Thus, the biphosphonate may be
(4-amino-1-hydroxybutylidene)bisphosphonate (alendronate),
(1-hydroxyethylidene)bisphosphonate (etidronate), and
[1-hydroxy-2-(3-pyridinyl)ethylidene]bisphosphonate (risedronate).
The preferred biphosphonate is alendronate.

Typically, the biphosphonate has a particle size of about 100 µm or less. Preferably, the biphosphonate has a particle size of about 5 µm to about 70 µm.

In order to be an effective effervescent composition, the composition according to the invention comprises an acid source or acid component. Typically, the acid source is an organic or mineral acid that is safe for consumption and which provides for effective and rapid effervescent disintegration upon contact with water (and the carbonate source to be discussed *infra*)*.*

The other component necessary for an effective effervescent composition is the carbonate source. The carbonate source should be safe for consumption and provide for effective and rapid effervescent disintegration upon contact with water and the acid source. Typically, the carbonate source is selected from the group consisting of carbonate salts, bicarbonate salts, and mixtures thereof. The carbonate salt can be any suitable carbonate salt and typically is selected from the group consisting of sodium carbonate, potassium carbonate, and combinations thereof. The bicarbonate salt can be any suitable bicarbonate salt and typically is selected from the group consisting of sodium bicarbonate, potassium bicarbonate, and combinations thereof.

The acid source and the carbonate source can be present in the effervescent composition according to the invention in any suitable amount. The relative amounts of acid source and carbonate source are such that the effervescent composition according to the invention, when dissolved in water produces a buffer solution. In this context, the term "solution" not necessarily implies that all components of the effervescent composition are dissolved. However, the acid source and the carbonate source will dissolve thereby forming a buffered solution having a pH-value in the range of about 3 to about 6.5, preferably about 3.5 to about 6, more preferably from about 4 to about 5.5.

Typically, the acid source is present in the effervescent composition according to the invention in an amount of about 25% by weight to about 75% by weight, based on the total weight of the composition. The carbonate source may be present in an amount of from about 20% to about 60%, based on the total weight of the composition.

In a preferred embodiment, the effervescent composition according to the invention may comprise the biphosphonate, the acid source comprising about 1% by weight to about 15% by weight citric acid (based on the total weight of the acid source and the carbonate source), and a carbonate source comprising about 0.1% by weight to about 10% by weight carbonate salt and about 25% to about 50% by weight bicarbonate salt (based on the total weight of the acid source and the carbonate source). This effervescent composition according to the invention, when dissolved in water, typically provides an aqueous composition having a buffered pH of about 3 to about 6.5.

This effervescent formulation according to the invention, which alleviates the need for humidity environmental monitoring during production, comprises magnesium sulfate (e.g. anhydrous) as humidity moderator.

The main object of the invention is to alleviate the need to control the environmental humidity condition during the production of a product. However, with the particular humidity-protection agent chosen in the present invention, it is possible to provide protection against humidity during the shelf life of the effervescent product when humidity may induce disintegration of the finished product. In order to achieve these results, it is suggested to provide the effervescent pharmaceutical composition according to the present invention with a molar ratio of the divalent cation divalent cation scavenger to the humidity moderator of at least 0.8/1 (i.e. determined as ratio fumaric acid to magnesium sulfate), but most preferably about 1/1. The ratio of the divalent cation scavenger to the humidity moderator may be in the range of 0.8/1 to 1.2/1.

According to a preferred aspect of the present invention, the overall molar concentration of the divalent cations provided by the humidity moderator or other components in the effervescent composition according to the invention is kept within the above range. Thus, the divalent cation scavenger may serve the purpose to scavenge or sequester any divalent cation in the effervescent composition. According to a particularly preferred aspect of the present invention, however, no additional source of magnesium or calcium is provided for in the composition - other than the humidity moderator.

The preferred effervescent pharmaceutical composition according to the invention comprises a carbonate source which may be selected from the group consisting of sodium dicarbonate, sodium carbonate, potassium dicarbonate, potassium carbonate, sodium glycine carbonate, anhydrous sodium dicarbonate, anhydrous sodium carbonate or a mixture thereof. A further important component of the effervescent composition is an acid source. The acid may be selected from the group consisting of citric acid, tartaric acid, malic acid, fumaric acid, adipic acid, succinic acid and anhydrous citric acid.

In a preferred embodiment, the acid source is citric acid (anhydrous) and the divalent cation scavenger is fumaric acid.

According to the invention, one may calculate the ratio of the divalent cation scavenger to the humidity moderator on the basis of the additional divalent cation scavenger only.

The humidity moderator is magnesium sulfate which may more preferably be present in an amount of 4 to 5.5 wt.%.

The divalent cation scavenger according to the invention is selected from the group consisting of citric acid, tartaric acid, malic acid, fumaric acid, adipic acid, and succinic acid. The preferred scavenger is fumaric acid.

Generally, the effervescent composition according to the invention may have a pleasing taste. Very often, active agents such as biphosphonates have an objectionable taste that should be masked by the formulation in which the active agents are delivered. Thus, it is preferred that the effervescent composition according to the invention comprises a sweetener, a flavorant, or a combination thereof. The sweetener can be any suitable material or artificial sweetener or a combination or natural and artificial sweeteners. Acceptable natural sweeteners include glucose, dextrose, invert sugar, fructose, and mixtures thereof. The natural sweetener may be present in the composition in an amount of about 10% by weight and above. Artificial sweeteners include saccharin, aspartame, chloro-derivatives of sucrose such as sucralose, cyclamate, acesulfame-K, sugar alcohols such as sorbitol, mannitol, and xylitol, and mixtures thereof. The artificial sweetener may be present in an amount of up to about 5 wt.%.

The favorant or flavoring agent can be a natural or a synthetic flavorant. Such flavoring agents include citrus oils such as lemon, orange, grape, lime, and grapefruit; fruit essences such as apple, a pear, a peach, a grape, a strawberry, a raspberry, cherry, plum, pineapple, and apricot and other fruit flavors. Useful flavorants include aldehydes and esters such as benzaldehyde, cherry, almond), citral (lemon, lime), neral (lemon, lime), decanal (orange, lemon), C₈-aldehyde (citrus fruits), C₉-aldehyde (citrus fruits), C₁₂-aldehyde (citrus fruits), and mixtures thereof. The preferred flavoring agent include cherry, citrus (lemon), or orange. Typically, the flavoring agents are present in the effervescent composition according to the invention in an amount of about 10 mg to about 100 mg.

Typically, the effervescent compositions according to the present invention also comprise a lubricant. A lubricant may be applied to the composition in particular when the final product is an effervescent tablet. Lubricants include hydrogenated or partially hydrogenated vegetable oils such as corn oil, canola oil, cotton seed oil, sesame oil, soy bean oil, grape seed oil, sunflower oil, safflower oil, olive oil, peanut oil, and combination thereof. Lubricants may also include calcium stearate, magnesium stearate, zinc stearate, polyoxyethylene monostearate, talc, polyethylene glycol, sodium benzoate, sodium lauryl sulfate, magnesium lauryl sulfate, light mineral oil, and combinations thereof.

The effervescent compositions according to the invention may further comprise a disintegrant to enhance the disintegration of a compressed tablet in water. Any suitable disintegrant may be used. Exemplary disintegrants include starch, alginic acid, guar gum, kaolin, bentonite, sodium starch glycolate, isoamorphous silicate, and microcrystalline cellulose.

According to a further embodiment of the invention, the effervescent pharmaceutical composition further comprises a lubricant, binders and an agent selected from the group of flavoring agents, colorants, and sweeteners. Preferred binders are selected from the group consisting of mannitol, lactose, dextrose, sorbitol, polyvinyl pyrrolidone, sodium chloride, sodium benzoate, lactose monohydrate or mixtures thereof. The lubricant may be selected from the group consisting of stearic acid salts, sodium benzoate, sodium lauryl sulfate and polyethylene glycol. The agent mentioned above may be selected from the group consisting of flavoring agents, colorants and sweeteners. The flavoring agent may be a lemon flavor. A preferred sweetener may be sodium cyclamate, sodium saccharinate or mixtures thereof.

In another aspect of the present invention, a process for preparing an effervescent composition is provided wherein the components of the effervescent composition as discussed above are admixed and further processed into tablets or a granulate.

A preferred process comprises the following steps:
a) Preparing of granules comprising the biphosphonate or its pharmaceutically acceptable salt and a binder;
b) Mixing the granules obtained in step a) with a mixture comprising of another binder, an acid source and a divalent cation scavenger;
c) Mixing a carbonate source, a lubricant, agents selected from the group of flavoring agents, colorants and sweeteners and the humidity moderator;
d) Mixing together the resulting mixtures of step (b) and (c) and adding another agent selected from the group of flavoring agents, colorants and sweeteners;
e) Pressing the resulting granules of step (d) into tablets or packaging them in sachets.

### Examples

### Example 1: Pharmaceutical composition (molar ratio divalent cation scavenger to humidity moderator 1:1)

| | Optimal quantity [mg] | Tablet weight [%] | Acceptable Range [%] |
|---|---|---|---|
| Sodium Alendronate* | 91.35 | 4.05 | 3.0-5.0 |
| Lactose Monohydrate | 31.00 | 1.37 | 1.0-2.0 |
| Polyvinyl pyrrolidone | 8.00 | 0.35 | 0.3-0.4 |
| Citric acid anhydrous | 400.00 | 17.71 | 16.0-19.0 |
| Sodium bicarbonate anhydrous | 550.00 | 24.36 | 23.0-25.5 |
| Sodium carbonate anhydrous | 110.00 | 4.87 | 4.0-5.5 |
| Lemon flavor | 25.00 | 1.11 | 1.0-2.0 |
| Sorbitol | 771.12 | 35.41 | |
| Sodium saccharinate | 15.00 | 0.66 | 0.5-0.7 |
| Sodium benzoate | 10.00 | 0.44 | 0.4-0.5 |
| Sodium Cyclamate | 10.00 | 0.44 | 0.4-0.5 |
| Magnesium sulfate (anhydrous) | 120.37 | 0.89 | 4.0-5.5 |
| Fumaric acid | 116.07 | 0.86 | |

| | | | |
|---|---|---|---|
| * corresponding to 70 mg Alendronic add | | | |

### Example 2: Preparation process

The process of preparation of an effervescent granule according to the invention is preferably performed according to the following steps:
a) Preparation of granules comprising sodium alendronate and lactose monohydrate, which are prepared with the aid of a solution consisting of polyvinyl pyrrolidone and deionized water, already pre-warmed at 70°C.
b) Mixing the granules obtained in step a) with a mixture comprising sorbitol, anhydrous citric acid and fumaric acid.
c) Mixing anhydrous sodium bicarbonate, magnesium sulfate, anhydrous sodium carbonate, sodium benzoate, sodium saccharinate and sodium cyclamate.
d) Mixing together the resulting mixtures of step (b) and (c) and adding lemon flavor.
e) The resulting granules of step (d) may either be packaged in sachets or pressed into tablets, which are finally packaged in a PVC/Alu blister.

## Claims

1. An effervescent pharmaceutical composition comprising a biphosphonate compound or its pharmaceutically acceptable salt, a carbonate source, an acid source, magnesium sulfate as humidity moderator and a divalent cation scavenger, wherein the divalent cation scavenger is selected from the group consisting of citric acid, tartaric acid, malic acid, fumaric acid, adipic acid, and succinic acid, wherein the molar ratio of divalent cation scavenger to humidity moderator is at least 0.8/1.

2. The effervescent composition according to claim 1, wherein the molar ratio of divalent cation scavenger to humidity moderator is about 1/1.

3. The effervescent composition according to claim 1 or 2, wherein the humidity moderator is present in an amount of 4 to 5,5 weight-%.

4. The effervescent composition according to any one of the preceding claims, wherein the divalent cation scavenger is fumaric acid.

5. The effervescent composition according to any one of the preceding claims, wherein the biphosphonate compound is alendronate or sodium alendronate.

6. The effervescent composition according to any one of the preceding claims, wherein the binder is selected from the group consisting of mannitol, lactose, dextrose, sorbitol, polyvinyl pyrrolidone, sodium chloride, sodium benzoate, lactose monohydrate or mixtures thereof; the lubricant is selected from the group consisting of stearic acid salts, sodium benzoate, sodium lauryl sulfate and polyethylene glycol, and the agent is selected from the group of flavoring agents, colorants and sweeteners, which are selected from the group consisting of lemon flavor, sodium cyclamate, sodium saccharinate or mixtures thereof.

7. A process for preparing an effervescent composition according to any one of the preceding claims, wherein the components of the composition are admixed and further processed into tablets or a granulate.

8. The process according to claim 7 comprising the following steps:
a) Preparing of granules comprising the biphosphonate compound or its pharmaceutically acceptable salt and a binder;
b) Mixing the granules obtained in step a) with a mixture comprising of another binder, an acid source and a divalent cation scavenger;
c) Mixing a carbonate source, a lubricant, agents selected from the group of flavoring agents, colorants and sweeteners and the humidity moderator;
d) Mixing together the resulting mixtures of step b) and c) and adding another agent selected from the group of flavoring agents, colorants and sweeteners;
e) Pressing the resulting granules of steps d) into tablets or packaging them into sachets.

## Patentansprüche

1. Efferveszierende pharmazeutische Zusammensetzung, umfassend eine Biphosphonatverbindung oder das pharmazeutisch akzeptable Salz davon, eine Carbonatquelle, eine Säurequelle, Magnesiumsulfat als Feuchtigkeitsmoderator und einen Fänger zweiwertiger Kationen, wobei der Fänger zweiwertiger Kationen aus der Gruppe gewählt ist, die aus Citronensäure, Weinsäure, Äpfelsäure, Fumarsäure, Adipinsäure und Bernsteinsäure besteht, wobei das Molverhältnis von Fänger zweiwertiger Kationen zu Feuchtigkeitsmoderator mindestens 0,8/1 beträgt.

2. Efferveszierende Zusammensetzung gemäß Anspruch 1, wobei das Molverhältnis von Fänger zweiwertiger Kationen zu Feuchtigkeitsmoderator etwa 1/1 beträgt.

3. Efferveszierende Zusammensetzung gemäß Anspruch 1 oder 2, wobei der Feuchtigkeitsmoderator in einer Menge von 4 bis 5,5 Gew.-% vorliegt.

4. Efferveszierende Zusammensetzung gemäß einem der vorhergehenden Ansprüche, wobei der Fänger zweiwertiger Kationen Fumarsäure ist.

5. Efferveszierende Zusammensetzung gemäß einem der vorhergehenden Ansprüche, wobei die Biphosphonatverbindung Alendronat oder Natriumalendronat ist.

6. Efferveszierende Zusammensetzung gemäß einem der vorhergehenden Ansprüche, wobei das Bindemittel aus der Gruppe gewählt ist, die aus Mannitol, Laktose, Dextrose, Sorbitol, Polyvinylpyrrolidon, Natriumchlorid, Natriumbenzoat, Laktosemonohydrat oder Mischungen davon besteht; das Gleitmittel aus der Gruppe gewählt ist, die aus Stearinsäuresalzen, Natriumbenzoat, Natriumlaurylsulfat und Polyethylenglykol besteht, und das Agens aus der Gruppe gewählt ist, die aus Aromastoffen, Färbemitteln und Süßstoffen besteht, die aus der Gruppe gewählt sind, die aus Zitronengeschmack, Natriumcyclamat, Natriumsaccharinat oder Mischungen davon besteht.

7. Verfahren für die Herstellung einer efferveszierenden Zusammensetzung gemäß einem der vorhergehenden Ansprüche, wobei die Bestandteile der Zusammensetzung beigemischt werden und weiter zu Tabletten oder einem Granulat verarbeitet werden.

8. Verfahren gemäß Anspruch 7, umfassend die folgenden Schritte:
a) Herstellen von Granulat umfassend die Biphosphonatverbindung oder das pharmazeutisch akzeptable Salz davon und ein Bindemittel;
b) Mischen des in Schritt a) erhaltenen Granulats mit einer Mischung, die sich aus einem weiteren Bindemittel, einer Säurequelle und einem Fänger zweiwertiger Kationen zusammensetzt;
c) Mischen einer Carbonatquelle, eines Gleitmittels, von Agentien, gewählt aus der Gruppe von Aromastoffen, Färbemitteln und Süßstoffen, und des Feuchtigkeitsmoderators;
d) Zusammenmischen der resultierenden Mischungen von Schritt b) und c) und Hinzufügen eines weiteren Agens, gewählt aus der Gruppe von Aromastoffen, Färbemitteln und Süßstoffen;
e) Pressen des resultierenden Granulats von Schritt d) zu Tabletten oder deren Verpacken zu Beuteln.

## Revendications

1. Composition pharmaceutique effervescente comprenant un composé biphosphonate ou son sel pharmaceutiquement acceptable, une source de carbonate, une source d'acide, du sulfate de magnésium comme modérateur d'humidité et un épurateur de cations divalents, dans laquelle l'épurateur de cations divalents est choisi parmi le groupe consistant en l'acide citrique, l'acide tartrique, l'acide malique, l'acide fumarique, l'acide adipique et l'acide succinique, dans laquelle le rapport molaire de l'épurateur de cations divalents sur le modérateur d'humidité est au moins 0,8/1.

2. Composition effervescente selon la revendication 1, dans laquelle le rapport molaire de l'épurateur de cations divalents sur le modérateur d'humidité est environ 1/1.

3. Composition effervescente selon la revendication 1 ou 2, dans laquelle le modérateur d'humidité est présent dans une quantité de 4 à 5,5 % en poids.

4. Composition effervescente selon l'une quelconque des revendications précédentes, dans laquelle l'épurateur de cations divalents est l'acide fumarique.

5. Composition effervescente selon l'une quelconque des revendications précédentes, dans laquelle le composé biphosphonate est un alendronate ou un alendronate sodique.

6. Composition effervescente selon l'une quelconque des revendications précédentes, dans laquelle le liant est choisi parmi le groupe consistant en le mannitol, le lactose, le dextrose, le sorbitol, la polyvinyle pyrrolidone, le chlorure de sodium, le benzoate de sodium, le lactose monohydraté ou des mélanges de ceux-ci ; le lubrifiant est choisi parmi le groupe consistant en des sels de l'acide stéarique, le benzoate de sodium, le laurylsulfate de sodium et le polyéthylène glycol, et l'agent est choisi parmi le groupe consistant en des agents aromatisants, des colorants et des édulcorants, qui sont choisis parmi le groupe consistant en un arôme citron, le cyclamate de sodium, le saccharinate de sodium ou des mélanges de ceux-ci.

7. Procédé de préparation d'une composition effervescente selon l'une quelconque des revendications précédentes, dans lequel les composants de la composition sont mélangés et ensuite transformés en comprimés ou en un granulat.

8. Procédé selon la revendication 7, comprenant les étapes suivantes:
a) préparation de granulés comprenant le composé biphosphonate ou son sel pharmaceutiquement acceptable et un liant;
b) mélange des granules obtenus à l'étape a) avec un mélange constitué d'un autre liant, d'une source d'acide et d'un épurateur de cations divalents;
c) mélange d'une source de carbonate, d'un lubrifiant, d'agents choisis parmi le groupe consistant en des agents aromatisants, des colorants et des édulcorants, et le modérateur d'humidité;
d) mélange ensemble des mélanges résultant des étapes b) et c) et ajout d'un autre agent choisi parmi le groupe consistant en des agents aromatisants, des colorants et des édulcorants;
e) pressage des granulés résultants de l'étape d) en comprimés ou emballage de ceux-ci en sachets.
